# EUROPEAN PATENT APPLICATION

(11) **EP 2 637 022 A1**
(43) Date of publication of application: **11.09.2013**
(21) Application number: 13001113.3
(22) Date of filing: 06.03.2013
(51) Int. Cl.: G01N 33/569

(54) **Method of inspecting periodontal disease causing bacteria using outer membrane vesicle and measuring appliance of the same**

(30) Priority: 06.03.2012 JP 2012049148
(71) Applicant: GC Corporation, Bunkyo-ku Tokyo 113-0033 (JP); Director-General of National Institute of Infectious Diseases, Tokyo (JP)
(72) Inventor: Nakao, Ryoma, Tokyo (JP); Senpuku, Hidenobu, Tokyo (JP); Ohnishi, Makoto, Tokyo (JP); Takayama, Kazuto, Tokyo (JP); Naito, Hiroki, Tokyo (JP); Sakuma, Tetsuo, Tokyo (JP)
(74) Representative: Müller-Boré & Partner Patentanwälte

(57) **Abstract**

The invention provides a method of inspecting bacteria by using an outer membrane vesicle of the bacteria, which is useful for easily diagnosing a periodontopathic bacteria with high precision. An outer membrane vesicle of bacteria to be detected is previously arranged in a measuring portion of a measuring appliance, a specimen material collected from a test subject is brought into contact with the measuring portion, and an anti-outer membrane vesicle antibody corresponding to the outer membrane vesicle in the specimen material is detected on the basis of an antigen antibody reaction. A measuring appliance for executing the inspecting method of the bacteria is structured such that the outer membrane vesicle of the bacteria to be detected is previously arranged in the measuring portion.

## Description

The present invention relates to a method of inspecting periodontal disease causing bacteria (hereinafter, refer simply to as periodontpathic bacteria) in saliva or the like using an outer membrane vesicle of the periodontopathic bacteria for diagnosing periodontal disease, and a measuring appliance to be used for the inspecting method.

As a countermeasure of a periodontal disease infection, there can be listed up preventative behaviors such as a plaque control on the basis of proper brushing of teeth or routine health checkups, and an improvement of dietary habit. However, from a practical standpoint, it has been an important point to find the periodontal disease infection progressing silently as accurately and early as possible, and carry out a medical treatment which is suitable for the symptom.

As a method of diagnosing periodontal disease, there has been known a method of detecting a specific arrangement existing in a gene of the periodontopathic bacteria by using an invader assay method (refer, for example, to patent document 1). The diagnosing method using the gene as mentioned above has a high specificity and can accurately measure the number of the bacteria, however, since it is necessary to temporarily send a specimen material such as saliva or the like to an inspection center and carry out an expert operation such as an extraction and an amplification of DNA at the inspection center, this method has a defect that it takes long and costs high.

Further, there has been a diagnosing method of analyzing enzymes from a gingival crevicular fluid by utilizing the fact that various enzymes are contained in the gingival crevicular fluid from a periodontal pocket (refer, for example, to patent document 2). However, since plural kinds of bacteria release the same enzyme, it has been impossible to accurately specify the kind of the bacterium. Further, in this method, since it is necessary to incubate for 5 to 30 minutes at a temperature between 50 and 60 °C, an expensive device and a plurality of operations have been necessary. Accordingly, it has been unrealistic to carry out this method in a general dental clinic or a group medical examination.

Further, there has been known the fact that an inflammation in a gum causes bleeding from a gingival sulcus or a periodontal pocket. There is a method of measuring a concentration of occult blood existing in saliva on the basis of a peroxidase-like reaction of a hemoglobin derived from the occult blood (refer, for example, to patent document 3). However, since a myeloperoxidase or a salivary peroxidase derived from a neutrophil universally exists in the saliva, the precision of the diagnosing method is not high. Further, there has been a problem that bleeding caused by an inspection such as a probing just before the diagnosis is detected.
Patent Document 1: Japanese Unexamined Patent Application Publication No. 2007-244349
Patent Document 2: Japanese Unexamined Patent Application Publication No. H2-261396
Patent Document 3: Japanese Unexamined Patent Application Publication No. 2002-181815

Accordingly, an object of the present invention is to provide a method of inspecting periodontopathic bacteria which can easily diagnose a periodontal disease with high precision, and a measuring appliance to be used for the inspecting method.

The inventors of the present invention devote themselves to make a study for solving the problem mentioned above. As a result, the inventors have paid attention to an outer membrane vesicle (hereinafter, refer simply to as OMV) holding an antigenicity, an immunogenicity and a pathogenicity of the periodontopathic bacteria, and have finished the present invention by inquiring into the fact that it is possible to diagnose existence and activity of target bacteria easily with high precision, by detecting an anti-OMV antibody in saliva and the like by using the OMV.

Namely, the present invention relates to a method of inspecting periodontopathic bacteria characterized by previously arranging an OMV of periodontopathic bacteria to be detected in a measuring portion of a measuring appliance, bringing a specimen material collected from a test subject into contact with the measuring portion, and detecting an anti-OMV antibody corresponding to the OMV in the specimen material on the basis of an antigen antibody reaction, and a measuring appliance for carrying out the inspecting method characterized in that the OMV of the periodontopathic bacteria to be detected is previously arranged in a measuring portion.

The inspecting method and the measuring appliance of the periodontopathic bacteria using the OMV according to the present invention is an excellent inspecting method and measuring appliance which can easily diagnose the periodontopathic bacteria with high precision.

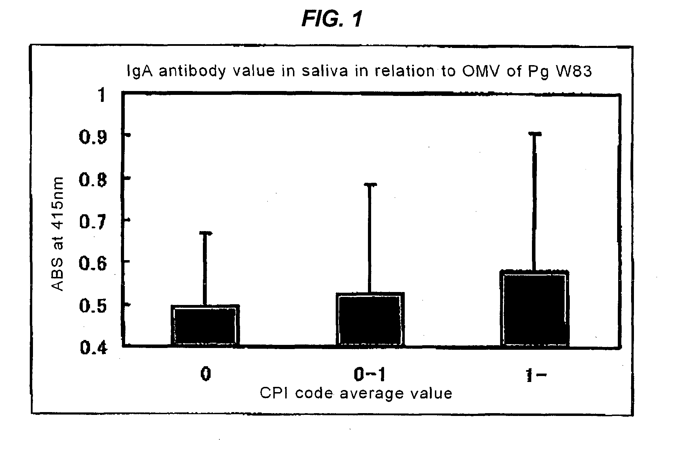

Fig. 1 is a view showing a result of measuring a human IgA antibody value in saliva in relation to an OMV of P. gingivalis strain W83 by utilizing an alkaline phosphatase modified anti-human IgA antibody.

The method of obtaining the OMV used in the present invention includes, for example, a method of culturing the periodontopathic bacteria to be inspected and centrifuging a culture supernatant thereof. In addition, there is a method of refining the culture supernatant by applying the culture supernatant to a micro filter. Specifically, the OMV can be obtained by carrying out a centrifugation of 2500 to 5000 x g for about 10 to 20 minutes, by suitable centrifuging condition for the periodontopathic bacteria to be inspected, collecting clear supernatant liquid, further carrying out a centrifugation of 70000 to 150000 x g for 1 to 4 hours, and removing the clear supernatant liquid.

As the periodontopathic bacteria to be inspected, for example, there are Porphyromonas gingivalis (hereinafter, refer to as P. gingivalis), Aggregatibacter actinomycetemcomitans, Treponema denticola, Tannerella forsythia, Prevotella intermedia, Fusobacterium nucleatum/periodonticum, Campylobacter rectus, Eikenella corrodens and the like. As the specimen material collected from the test subject, for example, there are urine, serum, saliva, gingival crevicular fluid, dental plaque and the like, as long as the specimen material having the target bacteria is appropriately collected. Among them, gingival crevicular fluid, dental plaque and saliva are preferable as the specimen material.

The measuring appliance in which the OMV of the bacteria to be detected is previously arranged can employ the conventional measuring appliance for detecting the antigen antibody reaction. For example, in an ELISA method, a hole bottom portion of a micro plate is set to a measuring portion of the anti-OMV antibody and the OMV is fixedly arranged. As a fixing method, the conventional antigen fixing method can be used without any special restriction, and it is possible to fix according to a method of suspending the OMV in a carbonate buffer, a PBS buffer, a TRIS buffer or the like, adding the solution to the hole bottom portion of the micro plate, and drying the liquid so as to physically adsorb, or a method of using a commercially available ELISA plate preparing kit.

The method of detecting the anti-OMV antibody according to the ELISA method includes a method of blocking the micro plate which the OMV is fixed to and modified in by the method mentioned above with a bovine serum albumin solution or a skim milk solution or the like, adding the specimen material, capturing the anti-OMV antibody in the specimen material on the hole bottom portion of the micro plate on the basis of the antigen antibody reaction, and removing the impurity by washing. Further, there is a method of detecting the antibody captured by the method mentioned above by coloring and fluorescing the antibody while using a commercially available enzyme labeled antibody which specifically recognizes the antibody and a reaction substrate thereof. Specifically, there are a method of using an anti-human immunoglobulin antibody modified by an alkaline phosphatase and a p-nitrophenyl sodium phosphate corresponding to the substrate so as to react, and thereafter measuring an absorbance by a plate reader, and a method of using an anti-human immunoglobulin antibody modified by peroxidases and 3,3',5,5'-tetramethyl benzidine (TMB) corresponding to the substrate so as to react, and thereafter measuring the absorbance by the plate reader.

Further, for example, in an immunochromatographic method, an arrangement is carried out by setting a place where the captured material is arranged by the conventional immunochromatographic method to the measuring portion of the anti-OMV antibody, and fixing the OMV within a porous body such as a membrane or the like. Specifically, a nitrocellulose membrane is used as the porous body, and a measuring portion preparing solution including the OMV is arranged on the nitrocellulose membrane by using an antibody applying device which can linearly arrange a small amount of liquid thereon so as to arrange the OMV.

The method of detecting the anti-OMV antibody according to the immunochromatographic method includes a method of applying the antigen antibody reaction to the OMV arranged in the detecting portion and the anti-OMV antibody in the specimen material, and detecting by a method of measuring a line which can be recognized by using the anti-immunoglobulin antibody modified by marker. As a specific modifying material, there can be utilized a dye such as a gold colloid, a platinum colloid, a latex bead or the like, and an enzyme such as an alkaline phosphatase, a peroxidases or the like in the case of being used together with a coloring substrate.

Further, for example, in an immunoprecipitation method, it is possible to utilize a carrier such as a bead generally used for recovering protein material as the measuring portion of the anti-OMV antibody. The bead includes several kinds of silica bead, latex bead and magnetic bead. A method of binding the OMV and the bead can utilize a generally known method such as a coupling reaction utilizing a functional group of the protein on the bead. The method of detecting the anti-OMV antibody in the specimen material purifies the anti-OMV antibody by adding the specimen material to a column having the bead therein, binding the bead and the anti-OMV antibody in the specimen material, and washing away the impurities by cleaning, and further obtains a solution including the only anti-OMV antibody by separating the binding between the bead and the anti-OMV antibody. An amount of the anti-OMV antibody in the solution can be measured and detected by using a protein determination method of a general method (a Bradford method, a BCA method or the like).

As a measuring appliance according to the present invention in which the OMV of the periodontopathic bacteria to be detected is previously arranged in the measuring portion, there can be listed up a micro plate which is used for an ELISA method. Specifically, it is the micro plate in which the OMV of the periodontopathic bacteria to be detected is previously fixed to and arranged in the hole bottom corresponding to the measuring portion. In the same manner, there can be listed up a micro flow path which has been used in the conventional ELISA method, and a measuring appliance in which the OMV is arranged in a measuring portion of a measuring appliance such as a capillary.

As the measuring appliance according to the present invention in which the OMV of the periodontopathic bacteria to be detected is previously arranged in the measuring portion, there can be listed up a device which is used in the immunochromatographic method. Specifically, there can be exemplified a measuring appliance structured such that in an immunochromatographic test device having an upper cover which is provided with a test solution dropping window and an inspection result detecting window, a lower cover which is provided with a test piece installation guide, and a porous piece which is stored between the upper cover and the lower cover and is porous and sheet like, the porous piece comprises a specimen material dropping portion provided on one end thereof in which a marker capable of detecting the antibody which specifically binds to the anti-OMV antibody included in the specimen material is attached, a measuring portion provided on the middle thereof in which the OMV which specifically binds to the anti-OMV antibody is previously arranged, and a specimen material absorbing portion provided on the opposite end to the specimen material dropping portion across the measuring portion.

A description will be in detail given below of the present invention by listing up an embodiment, however, the present invention is not limited to the following embodiment.

### <Preparation of OMV of periodontopathic bacteria>

P. gingivalis which is one of the periodontopathic bacteria was anaerobic cultured for two days by using a culture medium obtained by adding 1 µg/ml vitamin K and 5 µg/ml hemin to a Brain Heart Infusion (BHI) sterilized by autoclaving. A strain of P. gingivals employs W83. Only a culture supernatant was collected by centrifuging a culture solution, the cells were removed by using a 0.22 µm filter, an ultracentrifugation was carried out for 3 hours under condition of 100,000 x g, and a pellet was collected. The collected pellet was suspended by using 20 mM TRIS buffer and the OMV suspension was obtained. A protein abundance of the produced OMV was measured by the Bradford method.

### <Detection of anti-OMV antibody by ELISA method>

The OMV suspension regulated to 1 µg/ml was added at 100 µl to a 96-well plate, was sufficiently dried, and adsorbed to the bottom portion of the plate so as to be fixed.

After blocking the plate with 1% skim milk/PBS, the antigen antibody reaction was carried out by adding stimulated saliva collected by using a paraffin wax gum,

The anti-OMV antibody was marked by using an alkaline phosphatase modified anti-human IgA antibody, and a coloring reaction using disodium p-nitrophenyl phosphate was measured by the plate reader.

As a result, it was possible to recognize the matter that the anti-OMV antibody existing in saliva sample could be detected, and it was possible that a human IgA antibody value became greater in the case that an average value of a CPI code was higher, such as Fig. 1 showing the human IgA antibody value in the saliva in relation to the OMV of P. gingivalis strain W83. Therefore, there was clearly known that a titer of the anti-OMV antibody was associated at least with the CPI code. It is possible to carry out an inspection having a higher precision by evaluating while adding a combination with the other clinical symptom than the CPI code according to the same method.

### <Detection of anti-OMV antibody according to immunochromatographic method>

A nitrocellulose membrane (product name: HiFlow Plus HF120, manufactured by MILLIPORE company) was attached to a board having a thickness of 0.4 mm, and the board was cut at a length of 50 mm and a width of 5 mm. The OMV was arranged on the membrane by dropping 2 µl OMV suspension regulated at 25 µg/ml to the center portion of the membrane so as to form a circular shape and sufficiently drying. A sample pad having a length of 10 mm and made of a glass fiber was attached to one end of the membrane having the OMV arranged and formed a test end. Further, a water absorbing filter paper having a length of 30 mm was attached to an opposite end and a strip for immunochromatographic test was produced.

The test end of the strip for immunochromatographic test was inserted into the solution obtained by mixing 100 µl stimulated saliva diluted into fourfold by using a PBST buffer and 10 µl anti-human IgA antibody modified gold colloid suspension, and was checked by a visual observation after 15 minutes, and a determination was carried out by setting + in the case that a red circle coloring was seen, and setting - in the case that the red circle coloring was not seen.

As a result, it was recognized that the anti-OMV antibody could be detected by the immunochromatographic method as shown in Table 1. Further, it was possible to define a threshold value for detection by utilizing the result of the ELISA method, and it was suggested that the precision could be enhanced.

**Table 1**

| Sample ID | Result of immunochromatography | Result of ELISA |
|---|---|---|
| | W83 | W83 |
| 1 | - | 0.18 |
| 2 | - | 0.24 |
| 3 | - | 0.35 |
| 4 | - | 0.40 |
| 5 | - | 0.41 |
| 6 | - | 0.48 |
| 7 | - | 0.54 |
| 8 | - | 0.58 |
| 9 | + | 0.67 |
| 10 | + | 0.74 |
| 11 | + | 0.82 |
| 12 | + | 0.91 |

## Claims

1. A method of inspecting periodontopathic bacteria comprising the steps of:
previously arranging an outer membrane vesicle of a periodontopathic bacteria to be detected in a measuring portion of a measuring appliance;
bringing a specimen material collected from a test subject into contact with the measuring portion; and
detecting an anti-outer membrane vesicle antibody corresponding to said outer membrane vesicle in the specimen material on the basis of an antigen antibody reaction.

2. A measuring appliance comprising:
an outer membrane vesicle of a periodontopathic bacteria to be detected, the outer membrane vesicle being previously arranged in a measuring portion of the measuring appliance.

3. A measuring appliance according to claim 2, wherein said measuring appliance is a micro plate which said outer membrane vesicle is previously arranged in a hole bottom portion of the micro plate.

4. A measuring appliance according to claim 2, wherein said measuring appliance is an immunochromatographic test device having an upper cover which is provided with a test solution dropping window and an inspection result detecting window, a lower cover which is provided with a test piece installation guide, and a porous piece which is stored between the upper cover and the lower cover and is porous and thin, wherein said porous piece comprising:
a specimen material dropping portion provided on one end thereof in which a marker capable of detecting an antibody which specifically binds to an anti-OMV antibody included in the specimen material is attached,
a measuring portion provided on the middle thereof in which an OMV which specifically binds to the anti-OMV antibody is previously arranged,
and a specimen material absorbing portion provided on the opposite end to the specimen material dropping portion across the measuring portion.
